# EUROPEAN PATENT SPECIFICATION

(11) **EP 2 897 655 B1**
(45) Date of publication and mention of the grant of the patent: **02.11.2016**
(21) Application number: 13763251.9
(22) Date of filing: 17.09.2013
(51) Int. Cl.: A61L 27/06, A61L 27/10, A61L 27/30, A61L 27/56

(54) **SCAFFOLD WITH CORTICAL WALL**
GERÜST MIT KORTIKALER WAND
ÉCHAFAUDAGE AYANT UNE PAROI CORTICALE

(30) Priority: 18.09.2012 SE 1251041
(43) Date of publication of application: 29.07.2015
(73) Proprietor: Corticalis AS, 1450 Nesoddtangen (NO)
(72) Inventor: LYNGSTADAAS, S. Petter, 1450 Nesoddtangen (NO); ELLINGSEN, Jan Eirik, 1356 Bekkestua (NO); HAUGEN, Håvard J, 0376 Oslo (NO); TIAINEN, Hanna, 0858 Oslo (NO)
(74) Representative: Valea AB
(86) International application number: PCT/EP2013/069268
(87) International publication number: WO 2014/044672

(56) References cited:
- EP-A1- 1 764 116
- WO-A1-2011/007196
- WO-A2-2007/148240
- WO-A2-2008/078164
- US-A1- 2006 129 215
- HANNA TIAINEN ET AL: "Bone formation in TiObone scaffolds in extraction sockets of minipigs", ACTA BIOMATERIALIA, ELSEVIER, AMSTERDAM, NL, vol. 8, no. 6, 23 February 2012 (2012-02-23), pages 2384-2391, XP028480461, ISSN: 1742-7061, DOI: 10.1016/J.ACTBIO.2012.02.020 [retrieved on 2012-03-03]
- FOSTAD G ET AL: "Loadable TiO2 scaffolds-A correlation study between processing parameters, micro CT analysis and mechanical strength", JOURNAL OF THE EUROPEAN CERAMIC SOCIETY, ELSEVIER SCIENCE PUBLISHERS, BARKING, ESSEX, GB, vol. 29, no. 13, 1 October 2009 (2009-10-01), pages 2773-2781, XP026211305, ISSN: 0955-2219, DOI: 10.1016/J.JEURCERAMSOC.2009.03.017 [retrieved on 2009-04-24]
- HANNA TIAINEN ET AL: "Ultra-porous titanium oxide scaffold with high compressive strength", JOURNAL OF MATERIALS SCIENCE: MATERIALS IN MEDICINE, KLUWER ACADEMIC PUBLISHERS, BO, vol. 21, no. 10, 14 August 2010 (2010-08-14), pages 2783-2792, XP019856602, ISSN: 1573-4838, DOI: 10.1007/S10856-010-4142-1

## Description

### TECHNICAL FIELD

This document is directed to medical implants, in particular implants used to restore or replace bone tissue. The implant has a scaffold structure wherein at least part of the outer surface of the implant is provided with a nanoporous outer layer comprising titanium dioxide functioning as a barrier for soft tissue, such as epithelial tissue, growth into the scaffold.

### BACKGROUND OF THE INVENTION

Bone is made up of two types of tissue, cortical, or compact, bone and trabecular, or cancellous, bone. Cortical bone is a more dens structure, having a porosity of typically 5-30%. The cortical bone constitutes about 80% of the mass of bone. Trabecular bone is on the other hand much less dense and generally has a porosity of 30-90%.

Conditions such as trauma, tumours, cancer, periodontitis and osteoporosis may lead to bone loss, reduced bone growth and volume. For these and other reasons it is of great importance to find methods to improve bone growth and to regain bone anatomy. Scaffolds may be used as a framework for the cells participating in the bone regeneration process, but also as a framework as a substitute for the lost bone structure.

Orthopaedic implants are utilized for the preservation and restoration of the function in the musculoskeletal system, particularly joints and bones, including alleviation of pain in these structures. Orthopaedic implants are commonly constructed from materials that are stable in biological environments and that withstand physical stress with minimal deformation. These materials must possess strength, resistance to corrosion, have a good biocompatibility and have good wear properties. Materials which fulfil these requirements include biocompatible materials such as titanium and cobolt-chrome alloy.

Dental implants are utilized in dental restoration procedures in patients having lost one or more of their teeth. A dental implant comprises a dental fixture, which is utilized as an artificial tooth root replacement. Thus, the dental implant serves as a root for a new tooth. The dental implant is typically a screw, i.e. it has the shape of a screw, and it is typically made of titanium, a titanium alloy, zirconium or a zirconium alloy, The screw is surgically implanted into the jawbone, where after the bone tissue grows in close contact with the implant surface and the screw is thus fixated in the bone. This process is called osseointegration, because osteoblasts grow on and into the surface of the implanted screw, which becomes integrated with the bone, as measured at light microscopic level. By means of the osseointegration, a rigid installation of the screw is obtained.

For the purposes of tissue engineering it is previously known to use scaffolds to support growth of cells. It is believed that scaffold pore size, porosity and interconnectivity are important factors that influence the behaviour of the cells and the quality of the regenerated tissue. Prior art scaffolds are typically made of calcium phosphates, hydroxyl apatites and of different kinds of polymers.

One principle of tissue engineering is to harvest cells, expand the cell population *in vitro,* if necessary, and seed them onto a supporting three-dimensional scaffold, where the cells can grow into a complete tissue or organ. For most clinical applications, the choice of scaffold material and structure is crucial. In order to achieve a high cell density within the scaffold, the material needs to have a high surface area to volume ratio. The pores must be open and large enough such that the cells can migrate into the scaffolds. When cells have attached to the material surface there must be enough space and channels to allow for nutrient delivery, waste removal, exclusion of material or cells and protein transport, which is only obtainable with an interconnected network of pores. Biological responses to implanted scaffolds are also influenced by scaffold design factors such as three-dimensional microarchitecture. In addition to the structural properties of the material, physical properties of the material surface for cell attachment are essential.

Bone in-growth is known to preferentially occur in highly porous, open cell structures in which the cell size is roughly the same as that of trabecular bone (approximately 0.25-0.5 mm), with struts roughly 100 µm (0.1 mm) in diameter. Materials with high porosity and possessing a controlled microstructure are thus of interest to both orthopaedic and dental implant manufacturers. For the orthopaedic market, bone in-growth and on-growth options currently include the following: (a) DePuy Inc. sinters metal beads to implant surfaces, leading to a microstructure that is controlled and of a suitable pore size for bone in-growth, but with a lower than optimum porosity for bone in-growth; (b) Zimmer Inc. uses fibre metal pads produced by diffusion bonding loose fibres, wherein the pads are then diffusion bonded to implants or insert injection moulded in composite structures, which also have lower than optimum density for bone in-growth; (c) Biomet Inc. uses a plasma sprayed surface that results in a roughened surface that produces on-growth, but does not produce bone in-growth; and (d) Implex Corporation are using a chemical vapour deposition process to produce a tantalum-coated carbon microstructure that has also been called a metal foam. Research has suggested that this "trabecular metal" leads to high quality bone in-growth. Trabecular metal has the advantages of high porosity, an open-cell structure and a cell size that is conducive to bone in-growth. However, trabecular metal has a chemistry and coating thickness that are difficult to control. Trabecular metal is very expensive, due to material and process costs and long processing times, primarily associated with chemical vapour deposition (CVD). Furthermore, CVD requires the use of very toxic chemicals, which is disfavoured in manufacturing and for biomedical applications.

In order to ensure viable cell attachment, nutrient and waste product transportation, vascularisation, and passage of the newly formed bone tissue throughout the entire scaffold volume, a bone scaffold is required to have a well-interconnected pore network with large pore volume and an average pore connection size preferably exceeding 100 µm. In addition to the reticulated pore space, appropriate pore morphology and average pore size larger than 300 µm are necessary to provide adequate space and permeability for viable bone formation in a non-resorbable scaffold structure. However, one of the most important prerequisite for the scaffold structure is that the scaffold material itself is fully biocompatible and favours bone cell attachment and differentiation on its surface to promote the formation of a direct bone-to-scaffold interface.

Ceramic TiO₂ has been identified as a promising material for scaffold-based bone tissue repair, and highly porous TiO₂ scaffolds have previously been shown to provide a favourable microenvironment for viable bone ingrowth from surrounding bone tissue *in vivo.* The excellent osteoconductive capacity of these TiO₂ scaffolds has been attributed to the large and highly interconnected pore volume of the TiO₂ foam structure. However, as the mechanical properties of a scaffold are governed not only by the scaffold material but also by the pore architecture of the scaffold structure, increasing pore sizes and porosity are known to have a detrimental effect on the mechanical properties of cellular solids, and consequently reduce the structural integrity of the scaffold construct. As one of the key features of a bone scaffold is to provide mechanical support to the defect site during the regeneration of bone tissue, the lack of sufficient mechanical strength limits the use of the TiO₂ scaffold structure to skeletal sites bearing only moderate physiological loading. The mechanical properties of such ceramic TiO₂ foams should therefore be improved through optimized processing so as to produce bone scaffolds with adequate load-bearing capacity for orthopaedic applications without compromising the desired pore architectural features of the highly porous TiO₂ bone scaffolds.

Reticulated ceramic foams, such as those of WO08078164, have recently attracted increasing interest as porous scaffolds that stimulate and guide the natural bone regeneration in the repair of non-healing, or critical size, bone defects. Since the purpose of such a bone scaffold is to provide optimal conditions for tissue regeneration, the foam structure must allow bone cell attachment onto its surface as well as provide sufficient space for cell proliferation and unobstructed tissue ingrowth. Therefore, structural properties, such as porosity and pore morphology, of the 3D bone scaffold construct play a crucial role in the success of scaffold-based bone regeneration.

The mechanical properties of reticulated ceramic foams prepared by replication method are strongly dependent on the size and distribution of cracks and flaws in the foam structure, which typically determine the strength of the foam struts (Brezny et *al.* 1989). However, it has been an object in many studies to try to enhance the mechanical strength by optimising the various processing steps involved in the replication process.

A barrier membrane is a device that may be used on an implant to prevent epithelium, which regenerates relatively quickly, from growing into an area in which another, more slowly-growing tissue type, such as bone, is desired. Such a method of preventing epithelial migration into a specific area is known as guided tissue regeneration (GTR).

When barrier membranes are utilized, the superficial soft tissue flap remains separated from the underlying bone for the primary healing period and must survive on the vascular supply of the flap; it cannot rely on granulation tissue derived from the underlying bone.

Barrier membranes are typically used for two types of bony defects; space-making defects and non-space-making defects. Space-making defects, such as extraction sockets with intact bony walls, are not as demanding as non-space-making defects, such as sites of ridge augmentation, where there may be no support for the membrane and the soft tissue cover may cause collapse of the membrane during healing. Barrier membranes have been derived from a variety of sources, both natural and synthetic, and are marketed under various trade names.

The first membranes developed for this purpose were nonresorbable. Therefore, their use necessitates a second surgery for membrane removal some weeks after implantation. Historically, GTR and grafting techniques began with impractical millipore (paper) filter barriers. Expanded polytetrafluoroethylene (ePTFE) membranes were first used in 1984, being non-resorbable, but compatible with humans and not leading to infection. Although ePTFE is considered the standard for membranes and excellent outcomes have been achieved with this material, they are often contaminated with bacteria (which limits the amount of bone regrowth that will occur) and must eventually be removed via at least one extra surgery within 4-6 weeks after the tissue has regrown. Non-absorbable ePTFE membranes are still used clinically on a regular basis, and long-term studies suggest that bones regrown with ePTFE function as well as non-augmented naive bone.

The need for a second surgical procedure is of course a disadvantage associated with the use of these non-resorbable membranes, which led to the development of resorbable membranes.

Resorbable membranes are either animal-derived or synthetic polymers. They are gradually hydrolyzed or enzymatically degraded in the body and therefore do not require a second surgical step of membrane removal. Their sources are varied, beginning in early years with rat or cow collagen, cargile membrane, polylactic acid, polyglycolide, Vicryl, artificial skin and freeze-dried dura mater. Recently developed synthetic membranes often combine different materials.

Collagen resorbable membranes are of either type I or II collagen from cows or pigs. They are often cross-linked and take between four and forty weeks to resorb, depending on the type. Collagen absorbable barrier membranes do not require surgical removal, inhibit migration of epithelial cells, promote the attachment of new connective tissue, are not strongly antigenic and prevent blood loss by promoting platelet aggregation leading to early clot formation and wound stabilization. Collagen membranes may also facilitate primary wound closure via fibroblast chemotactic properties, even after membrane exposure. Compared to ePTFE membranes, resorbable barriers allow for fewer exposures and therefore reduce the effects of infection on newly formed bone, Use of collagen membranes in particular, with bone mineral as a support and space maintainer, has achieved predictable treatment outcomes. However, due to their animal origin, there is always a risk for allergic reactions when collagen membranes are used.

Synthetic resorbable membranes may be polymers of lactic acid or glycolic acid. Their ester bonds are degraded over 30-60 days, leaving free acids that may be inflammatory. The majority of studies consider synthetics at least comparable to other membranes like ePTFE and collagen. The integrity of resorbable membranes over the healing period has been questioned relative to the ePTFE membranes.

As is clear from the above, there still exists a need in the art for new structures which can function as barrier membranes.

The object of the present invention is to overcome or at least mitigate some of the problems associated with the prior art.

### SUMMARY OF INVENTION

One object of the present document is to provide a titanium dioxide scaffold suitable as a medical implant, which scaffold is provided with a nanoporous outer layer preventing soft tissue growth into the scaffold.

This object is obtained by the present disclosure which in one aspect is directed to a titanium dioxide scaffold, wherein at least part of the outer surface of the titanium dioxide scaffold is provided with a nanoporous outer layer comprising titanium dioxide, wherein the pores of the nanoporous outer layer have an average pore diameter of 1 nm-5000 nm.

The pores of the nanoporous outer layer have a diameter such that it prevents growth of soft tissue over it and into the titanium dioxide scaffold. Also, the nanoporous outer layer increases the strength of the scaffold as it has a reduced pore size as compared to the scaffold structure in itself. Further, as the nanoporous outer layer is an integral part of the scaffold, the nanoporous outer layer does not have to be removed nor does it degrade in a body, as compared to the non-resorbable and resorbable barrier membranes discussed above. Also, the nanoporous outer layer may have a beneficial effect on slowly growing osteoblast cells. Without wishing to be bound by theory, this may be due to the fact that the slowly growing osteoblast cells are given sufficient time to grow over the nanoporous outer layer as this is not degraded and/or that the nanoporous outer layer in itself has an osteoblast growth promoting effect.

The present document is also directed to a method for producing a titanium dioxide scaffold wherein at least part of the outer surface of the titanium dioxide scaffold is provided with a nanoporous outer layer comprising titanium dioxide, wherein the pores of the nanoporous outer layer have an average pore diameter of 1 nm-5000 nm, said method comprising or consisting of the steps of:
a) providing a titanium dioxide scaffold,
b) optionally coating at least part of the titanium dioxide scaffold with a titanium dioxide slurry,
c) optionally removing excess slurry from the titanium dioxide scaffold of step b),
d) providing a powder comprising titanium dioxide and at least one polymer onto at least a part of the outer surface of the titanium dioxide scaffold,
e) sintering the titanium dioxide scaffold of step d); and
f) optionally repeating steps b) through e).

In the above method, step b) may be preceded by providing a titanium dioxide slurry to at least a part of the titanium dioxide scaffold where the nanoporous outer layer is to be formed, followed by sintering the titanium dioxide scaffold. Alternatively, or in addition, step e) or f) in the above method may be followed by providing a titanium dioxide slurry to at least a part of the titanium dioxide scaffold where the nanoporous outer layer is to be formed, followed by sintering the titanium dioxide scaffold.

The present document is also directed to a titanium dioxide scaffold provided with a nanoporous outer layer comprising titanium dioxide obtainable or obtained by the above method.

Further, the present document is directed to a medical implant, such as an orthopaedic implant, comprising a titanium dioxide scaffold provided with a nanoporous outer layer comprising titanium dioxide, wherein the pores of the nanoporous outer layer have an average pore diameter of 1 nm-5000 nm. Also disclosed is the use of this scaffold or a medical implant comprising it for the regeneration, repair, substitution and/or restoration of tissue, such as bone or cartilage.

Other features and advantages of the invention will be apparent from the following detailed description, drawings, examples, and from the claims.

### DEFINITIONS

"Scaffold" in the present context relates to an open porous structure. By "titanium dioxide scaffold" is meant a scaffold comprising predominantly titanium dioxide as the building material for the scaffold structure (i.e. more than 50 wt% titanium dioxide, such as about 51 wt%, 60 wt%, 70 wt%, 80 wt%, 90 wt%, 95 wt%, 96 wt%, 97 wt%, 98 wt%, 99 wt% or 100 wt% titanium dioxide, such as about 51-100 wt%, 60-100 wt%, 60-90 wt%, 70-100 wt%, 70-90 wt%, 80-90 wt%, or 80-95 wt% titanium dioxide). The titanium dioxide scaffold may thus comprise or consist of titanium dioxide as the building material for the scaffold. The scaffold may in addition comprise other substances, such as a surface coating of biologically active molecules and/or the nanoporous outer layer.

"Fractal dimension strut" is a statistical quantity that gives an indication of how completely a fractal appears to fill space, as one zooms down to finer and finer scales. There are many specific definitions of fractal dimension and none of them should be treated as the universal one. A value of 1 pertains to a straight line. The higher the number the more complex is the surface structure. Fractal dimension is in the present document calculated using the Kolmogorov or "box counting" method (Larry S. *et a*/*.* 1989). It is calculated in both 2d and 3d in Skyscan CTAn, Kontich , Belgium. The surface or volume is divided into an array of equal squares or cubes, and the number of squares containing part of the object surface is counted. This is repeated over a range of box sizes such as 3-100 pixels. The number of boxes containing surface is plotted against box length in a log-log plot, and the fractal dimension is obtained from the slope of the log-log regression.

By "pore diameter" is in the context of the present document intended the hydraulic diameter of a pore without its surrounding walls. The hydraulic diameter is well known to the person skilled in the art and is defined as 4*area of a pore divided by the circumferential length of the pore.

"Total porosity" is in the present context defined as all compartments within a body which is not a material, i.e. the space not occupied by any material. Total porosity involves both closed and open pores.

By "inner strut volume" is meant the volume of the inner lumen of the strut.

By "sintering", "sinter" and the like is meant a method for making objects from powder, by heating the material (below its melting point) until its particles adhere to each other (fuse). Sintering is traditionally used for manufacturing ceramic objects, and has also found uses in such fields as powder metallurgy.

A "medical prosthetic device, "medical implant", "implant" and the like in the present context relates to a device intended to be implanted into the body of a vertebrate animal, such as a mammal, e.g. a human mammal. Implants in the present context may be used to replace anatomy and/or restore any function of the body. Examples of such devices include, but are not limited to, dental implants and orthopaedic implants. In the present context, orthopaedic implants includes within its scope any device intended to be implanted into the body of a vertebrate animal, in particular a mammal such as a human, for preservation and restoration of the function of the musculoskeletal system, particularly joints and bones, including the alleviation of pain in these structures. In the present context, dental implants include any device intended to be implanted into the oral cavity of a vertebrate animal, in particular a mammal such as a human, in tooth restoration procedures. Generally, a dental implant is composed of one or several implant parts. For instance, a dental implant usually comprises a dental fixture coupled to secondary implant parts, such as an abutment and/or a dental restoration such as a crown, bridge or denture. However, any device, such as a dental fixture, intended for implantation may alone be referred to as an implant even if other parts are to be connected thereto. Orthopaedic and dental implants may also be denoted as orthopaedic and dental prosthetic devices as is clear from the above.

In the present context, "subject" relate to any vertebrate animal, such as a bird, reptile, mammal, primate and human.

By ceramics are in the present context meant objects of inorganic powder material treated with heat to form a solidified structure.

By "soft tissue" is in the context of the present document intended tissues that connect, support, or surround other structures and organs of the body, not being bone. Soft tissue includes ligaments, tendons, fascia, skin, fibrous tissues, fat, synovial membranes, epithelium, muscles, nerves and blood vessels.

By "hard tissue" is in the context of the present document intended mineralized tissues, such as bone and teeth, and cartilage. Mineralized tissues are biological tissues that incorporate minerals into soft matrices.

### BRIEF DESCRIPTION OF DRAWINGS

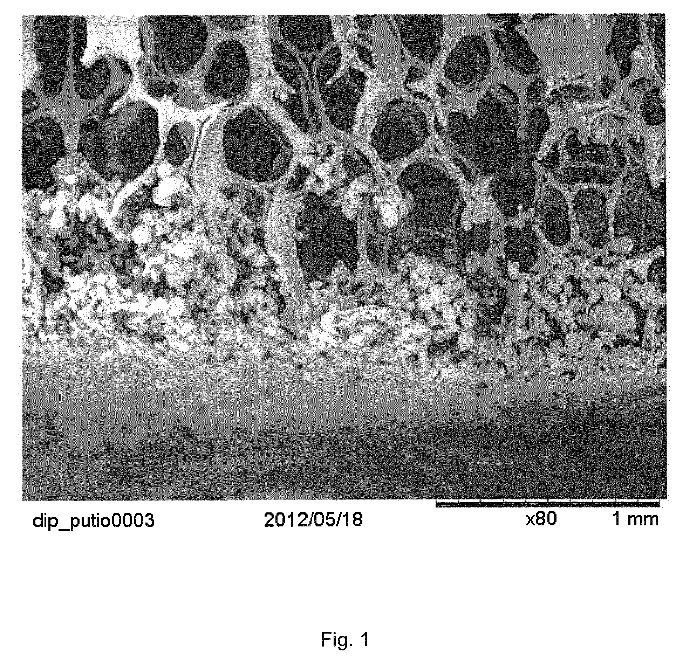

Figure 1: SEM image of a nanoporous outer layer on the outer surface of a titanium dioxide scaffold. The nanoporous outer layer is the granulated structure in the lower part of the image. The titanium dioxide scaffold with a nanoporous outer layer was produced by dipping a titanium dioxide scaffold in a dry powder of titanium dioxide (Kronos) and a polyethylene polymer powder in a ratio 1:10 by weight followed by sintering at 2.5 hours at 1500°C.

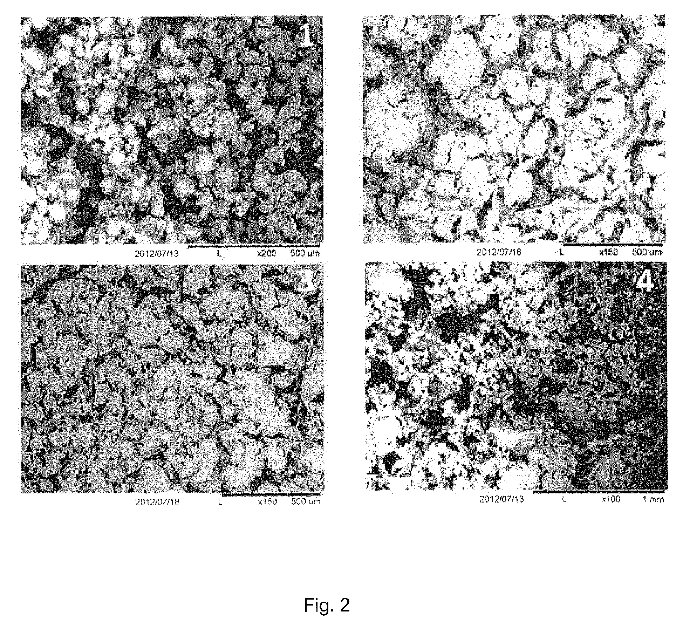

Figure 2: SEM images of nanoporous outer layer (cortical wall) after different procedures according to Example 2: 1) Dipping in dry TiO₂ and polymer powder followed by sintering, 2) Dipping in dry TiO₂ and polymer powder followed by sintering before dipping in dense TiO₂ slurry and sintering, 3) Dipping in pressed dry TiO₂ and polymer powder followed by sintering before dipping in dense TiO₂ slurry and sintering, 4) dipping in dense TiO₂ slurry and sintering followed by dipping in dry TiO₂ and polymer powder.

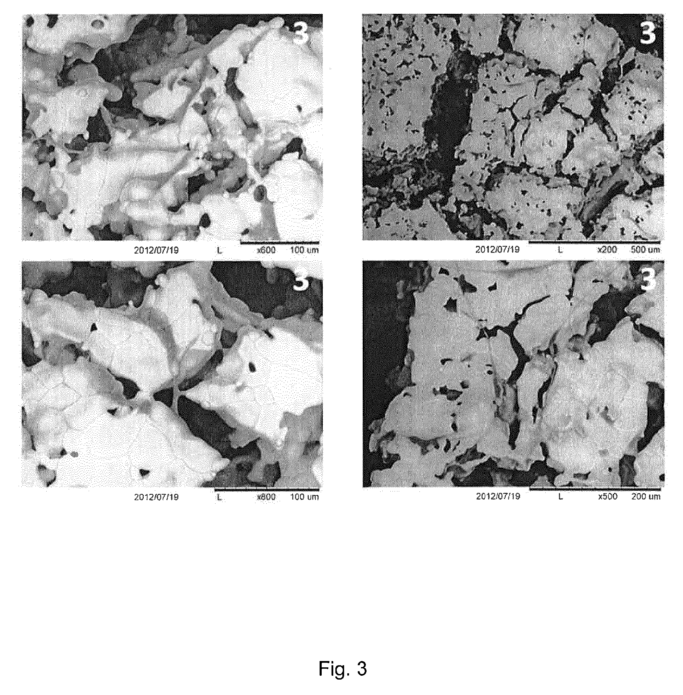

Figure 3: SEM image of cortical wall (nanoporous outer layer) on titanium dioxide scaffold with seeded osteoblasts after seven days of culturing in culture medium. Human osteoblast were seeded at a concentration of 20 000 cells per mL dropwise onto the cortical wall, placed in an incubator at 37°C.

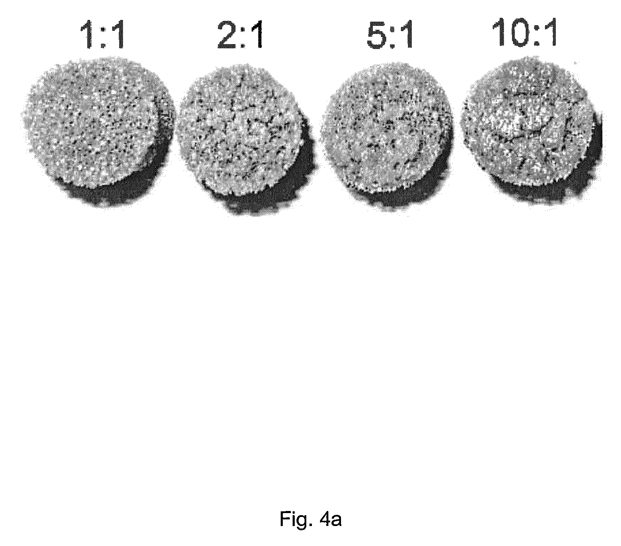

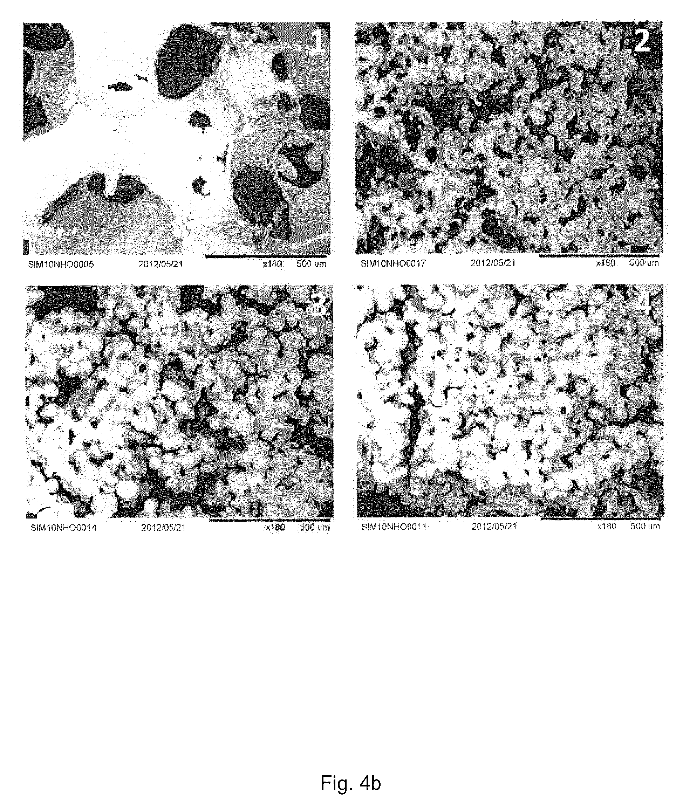

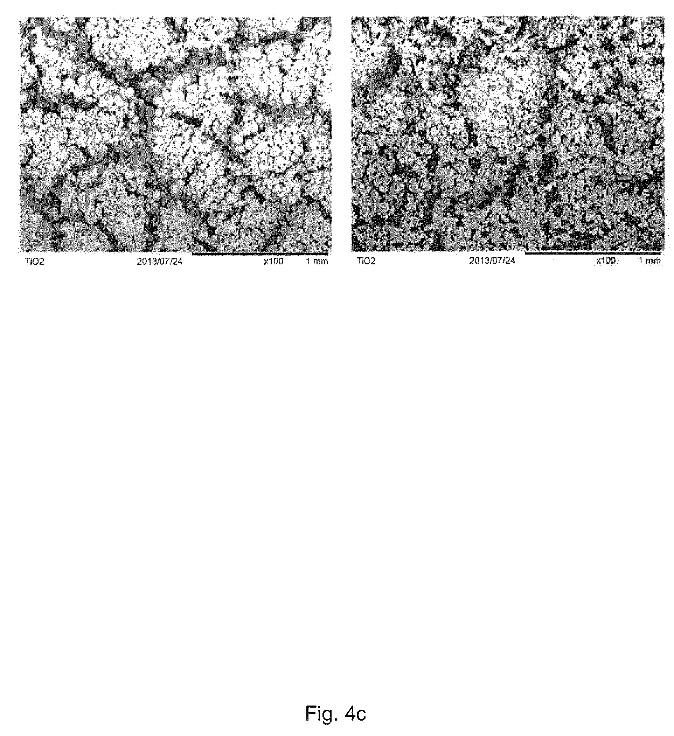

Figure 4: Fig. 4 a: The appearance of cortical wall structures prepared with varying TiO₂-to-polymer particle ratio. Fig. 4b: The morphology of cortical wall structures prepared with varying TiO₂-to-polymer particle ratio. 1) 1:1, 2) 2:1, 3) 5:1, 4) 10:1. Fig.4c: The morphology of cortical wall structures prepared 1) without PE particles and 2) with PE particles as porogen (TiO₂-to-PE particle ratio 10:1).

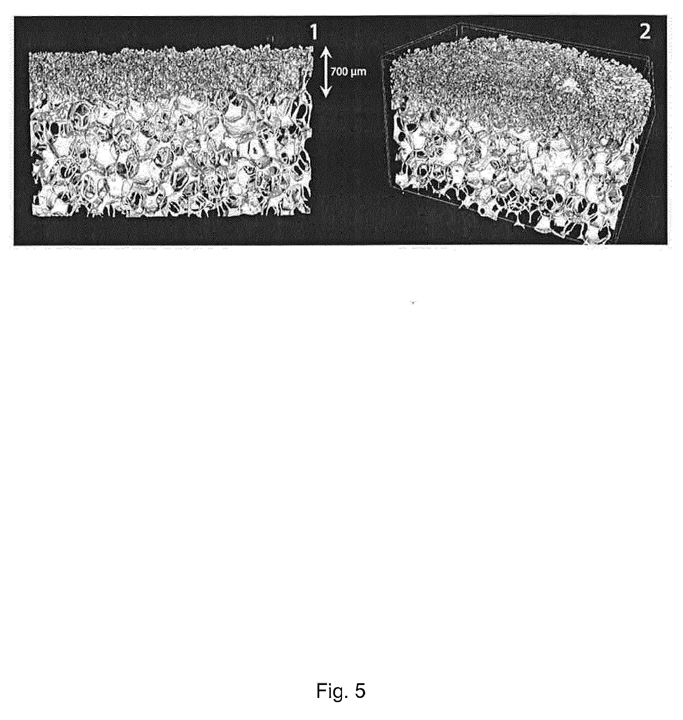

Figure 5: Cortical wall structure prepared using a TiO₂-to-polymer particle ratio 10:1. 1) Cross-sectional image displaying the uniform and homogenously distributed nano- and micropore network that was formed in the cortical wall layer structure of approximately 700 µm thickness. 2) Three-dimensional appearance of a TiO₂ scaffolds with an incorporated cortical wall structure.

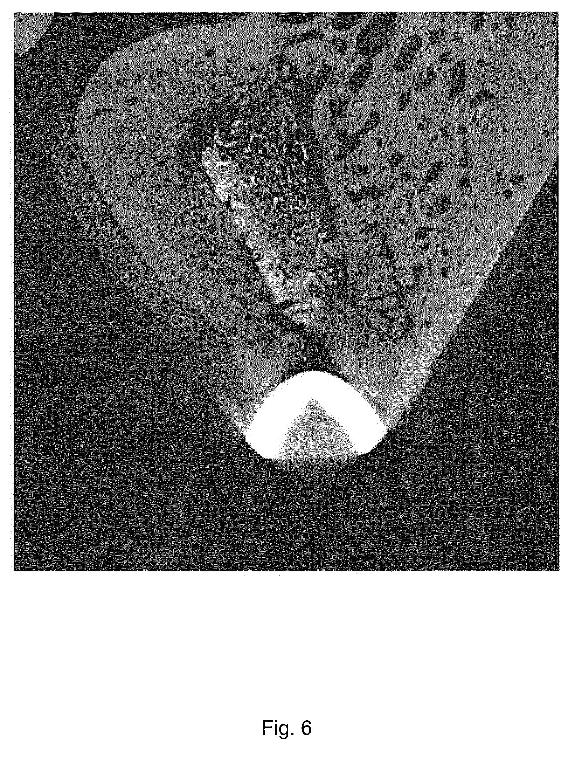

Figure 6: Bone formation on titanium dioxide scaffold with cortical wall after implantation. After six months of healing there was substantially more bone on top of the cortical wall (in comparison to sham), where one can see a thick wall of newly formed bone on top of the cortical wall.

### DETAILED DESCRIPTION OF THE INVENTION

This disclosure is directed to a titanium dioxide (TiO₂) scaffold having a soft tissue barrier on at least part of its outer surface in the form of a nanoporous outer layer comprising titanium dioxide wherein the pores in the nanoporous outer layer have an average pore diameter of 1 nm-5000 nm. By "nanoporous outer layer" is therefore in the present context meant a porous layer comprising or consisting of titanium dioxide wherein the average pore diameter of the pores in the porous layer is 1 nm-5000 nm. Other typical features of the nanoporous outer layer, such as thickness, porosity etc., are disclosed elsewhere in this document. Also disclosed is a method for producing a titanium dioxide scaffold with such a nanoporous outer layer. The nanoporous outer layer at least substantially prevents the ingrowth of soft tissue, such as epithelial tissue into the scaffold. In the present context this nanoporous outer layer comprising titanium dioxide wherein the pores in the nanoporous outer layer have an average pore diameter of 1 nm-5000 nm may therefore be denoted a "cortical wall section", "cortical wall", "nanoporous outer layer", or a "soft tissue barrier". The nanoporous outer layer's structure mimics natural cortical bone. Due to the nanoporous outer layer, the mechanical strength of the titanium dioxide scaffold is also increased as the nanoporous outer layer is stronger than the titanium dioxide scaffold in itself due to the smaller pore diameter of the nanoporous outer layer as compared to the pore diameter of the titanium dioxide scaffold structure. In addition, the titanium dioxide material of the nanoporous outer layer may promote osteoblasts to grow on the nanoporous outer layer surface. These effects will be described in more detail below. The titanium dioxide scaffold provided with the nanoporous outer layer as disclosed herein may be denoted a "cortical wall titanium dioxide scaffold".

The present document discloses a titanium dioxide scaffold, wherein at least part of the outer surface of the titanium dioxide scaffold is provided with a nanoporous outer layer comprising titanium dioxide, wherein the pores of the nanoporous outer layer have an average pore diameter of 1 nm-5000 nm. However, the average pore diameter of the pores in the nanoporous layer may also be about 10 nm-1000 nm, such as 10 nm-500 nm, 50 nm-200 nm or 50 nm-100 nm. Typically, the nanoporous outer layer consists of titanium dioxide. This document is also directed to a nanoporous outer layer comprising titanium dioxide as disclosed herein as such. The nanoporous outer layer may e.g. be produced by the method disclosed elsewhere in this document.

The total porosity of the nanoporous outer layer is typically about 1-50%, such as 3-30%, 5-30% or 5-10%. The porosity of the nanoporous outer layer is therefore typically close to the one of natural cortical bone, which generally has a porosity of 5-30% or 5-10%. In the context of the present document, it is important to note that the nanoporous outer layer has a pore size, pore architecture and/or porosity that differs from the pore size, pore architecture and/or porosity of the titanium dioxide scaffold structure itself.

The pore diameter of the nanoporous outer layer is selected to allow small objects, such as nutrients, ions and fluids, to pass through the nanoporous outer layer and enter the scaffold. However, the diameter is also selected so that larger objects (e.g. larger than 5 µm in diameter), such as cells, cannot penetrate the nanoporous outer layer, which therefore functions as a barrier for cells (such as the resorbable and non-resorbable barrier membranes disclosed elsewhere herein). Soft tissue cells will therefore substantially not grow through or into the nanoporous outer layer. However, osteoblasts may grow over, but not into, the nanoporous outer layer. Without wishing to be bound by theory, this may be due to a positive effect on osseointegration by the nanoporous outer layer as this is made of titanium dioxide (which is known to have such an effect). Thereby, when the scaffold is implanted in bone, the scaffold may be more or less fully encapsulated in bone tissue.

As compared to resorbable and non-resorbable membranes disclosed elsewhere herein, the nanoporous outer layer is an integral part of the titanium dioxide scaffold. Therefore, the need for a separately provided extra membrane is avoided and instead a "barrier" firmly attached to the scaffold is provided. However, in comparison to non-resorbable membranes, the nanoporous outer layer does not need to be removed after fulfilling its function as a cell barrier. Also, in contrast to the resorbable membranes, the nanoporous outer layer remains on the scaffold and is not intended to be degraded over time. As disclosed elsewhere herein, this may have a beneficial effect on bone growth, allowing bone to grow over the surface of the nanoporous outer layer. Further, as the nanoporous outer layer is not degraded over time, there will be no potentially harmful degradation products released at the implantation site. In comparison, when a resorbable membrane is used, this is broken down, typically leaving degradation products such as carbon dioxide, acids and the like which may cause inflammation and interfere with tissue healing. This disadvantage does not occur with the nanoporous outer layer disclosed herein.

The nanoporous outer layer typically has a thickness of 10-1000 µm, such as 50-500 µm, 75-200 µm, 50-100 µm, 300-1000 µm, or 500-900 µm. As may be seen in Fig. 1, the nanoporous outer layer is situated on the outer surface of the titanium dioxide scaffold but to some degree also extends into the most outer parts of the pores of the scaffold. However, the nanoporous outer layer does not extend into and coat the more inner parts of the scaffold. The nanoporous outer layer is thereby firmly attached to the scaffold which reduces the risk that it will flake off. The nanoporous outer layer is therefore integrated in the scaffold. Thus, the nanoporous other layer may not easily be removed from the scaffold in contrast to the resorbable and non-resorbable barrier membranes. Still, the nanoporous outer layer forms a well-defined layer on the scaffold's outer surface (see e.g. Fig. 1).

The nanoporous outer layer may be provided on the outer surface of any titanium dioxide scaffold in order to provide the scaffold with a barrier mimicking natural cortical bone. Depending on the type and intended function of the titanium dioxide scaffold, the nanoporous outer layer may be provided on a smaller or a larger part of the outer surface of the scaffold. Generally, only a part of the outer surface of the titanium dioxide scaffold is provided with the nanoporous outer layer as it often is desirable to have at least part of the scaffold structure open for events such as cell in-growth (e.g. by bone cells), nutrient and waste product transportation, vascularisation, and passage of newly formed bone tissue throughout the entire scaffold volume. Therefore, typically about 1-99%, 5-80%, 5-50%, 5-30% or 5-10% of the outer surface of the titanium dioxide scaffold is covered by the nanoporous outer layer. Of course the nanoporous outer layer may be provided on one or more different part(s) of the scaffold. Intentionally, or typically, the nanoporous layer is provided on a part of the scaffold surface that will be indirect contact with soft tissue cells when implanted into a body.

The nanoporous outer layer provides an additional stability (strength) to the titanium dioxide scaffold due to its dense structure mimicking the structure of cortical bone. The more of the scaffold surface that is covered by the nanoporous outer layer, the more pronounced this effect is. The nanoporous other layer may therefore be used for increasing the strength of a titanium dioxide scaffold. However, as mentioned above, it may be preferred that not the entire outer surface of the titanium dioxide scaffold is covered by the nanoporous outer layer.

Further, the nanoporous outer layer forms a barrier on the surface of the scaffold. This barrier prevents or reduces the growth of epithelial tissue on and into the scaffold. Thereby, more slowly growing tissue has a better opportunity for growing onto the scaffold (from parts of it not coated with the nanoporous outer layer) without epithelial tissue already blocking the pores of the scaffold.

Another advantage with the titanium dioxide scaffold having a nanoporous outer layer as disclosed herein, is that the nanoporous outer layer, containing the titanium dioxide ceramic, is so strong that it allows drilling through it without breaking (such as when a screw is to be fixed to the scaffold, e.g. during lateral or ridge augmentation).

### The titanium dioxide scaffold

The titanium dioxide scaffold of the present document is a reticulated scaffold which may function as a structural support which allows tissue formation by creating a three dimensional space for cellular attachment and ingrowth. The titanium dioxide of the scaffold provides a scaffold which is biocompatible and which can be processed into different shapes to provide mechanical support and a framework for cellular growth. Thus, the titanium dioxide scaffold provided with the nanoporous outer layer provides a suitable structure to be used in tissue engineering, such as for regeneration of bone.

The titanium dioxide scaffold suitable for being provided with a nanoporous outer layer as disclosed herein is a scaffold basically formed of titanium dioxide, i.e. titanium dioxide is the main structural component of the titanium dioxide scaffold. The titanium dioxide scaffold should adopt an open porous structure.

However, the titanium dioxide scaffold may be coated with different kinds of coatings, such as a coating comprising biomolecules (see below). Still, typically, titanium dioxide is the main structural component responsible for making up the scaffold structure. The titanium dioxide scaffold may also consist of titanium dioxide.

Typically, the titanium dioxide scaffold is produced by a method of dipping a combustible porous structure, such as a polymer sponge structure, in a titanium dioxide slurry, allowing the slurry to solidify on the sponge and performing one or more sintering steps to remove the sponge and create a strong scaffold structure (see e.g. the methods disclosed in WO08078164).

The titanium dioxide scaffold typically is a macroporous scaffold comprising macropores and interconnections. Macropores of the titanium dioxide scaffold have a pore diameter in the range between approximately 10-3000 µm, such as 20-2000 µm, about 30-1500 µm or about 30-700 µm. It is important that the titanium dioxide scaffold allows for the ingrowth of larger structures such as blood vessels and trabecular bone, i.e. also comprises pores of about 100 µm or more. It is important that at least some of the pores are interconnected and/or partially interconnected. In contrast, the pores of the nanoporous outer layer are much smaller, therefore not allowing ingrowth of cells. Thus, cells will grow into the titanium dioxide scaffold from the parts of the scaffold onto which the nanoporous outer layer is not provided.

The pore diameter may affect the rate and extent of growth of cells into the titanium dioxide scaffold and therefore the constitution of the resulting tissue. The macroporous system typically occupies at least 50% volume of the titanium dioxide scaffold. The volume of the macro- and micropores in the titanium dioxide scaffolds may vary depending on the function of the titanium dioxide scaffold. If the aim with a treatment is to replace much bone structure and the titanium dioxide scaffold can be kept unloaded during the healing time, the titanium dioxide scaffold may be made with a macroporous system occupying up to 90% of the total scaffold volume.

The titanium dioxide scaffold typically has a total porosity of about 40-99%, such as 70-90%, e.g. 80-90%.

The fractal dimension strut of the titanium dioxide scaffold is typically about 2.0-3.0, such as about 2.2-2.3. The strut thickness affects the strength of the titanium dioxide scaffolds, the thicker the struts in the titanium dioxide scaffold are, the stronger the titanium dioxide scaffold is.

The titanium dioxide scaffold typically has an inner strut volume of about 0.001-3.0 µm³, such as about 0.8-1.2 µm³ A lower volume and a higher fractal number give a stronger scaffold.

It will be understood by those of skill in the art that the titanium dioxide scaffold also has a structure on the microlevel and the nanolevel. This micro and nano structure may be modified due to the manufacturing conditions. The pore diameters on the microlevel are typically in the range of 1-10 µm. The pores on the nanolevel typically are less than 1 µm in diameter. It is important to note that the scaffold also has a macroporous structure with pore diameters in the magnitude of about 100 µm which allows for the ingrowth of cells.

A titanium dioxide scaffold in the present context (without the nanoporous outer layer) typically has a combined micro and macro pore diameter of approximately 10- 3000 µm, such as 20-2000 µm, 30-1500 µm or 30-700 µm. The pore diameter may also be above 40 µm, with interconnective pores of at least 20 µm.

The size and the shape of the titanium dioxide scaffold are decided depending on its intended use. The titanium dioxide scaffold size and shape may be adjusted either at the stage of production or by later modification of a ready scaffold. The titanium dioxide scaffolds may therefore easily be tailored for their specific use in a specific subject.

The titanium dioxide scaffold may for example be a titanium dioxide scaffold as disclosed in WO08078164.

Also, biomolecules may be provided to the surface of the titanium dioxide scaffold. If biomolecules are to be provided to the titanium dioxide scaffold, these may be provided after providing the scaffold with a nanoporous outer layer comprising titanium dioxide. The presence of biomolecules may further increase the biocompatibility of the titanium dioxide scaffold and rate of cell growth and attachment. Biomolecules comprise in the present context a wide variety of biologically active molecules including natural biomolecules (i.e. naturally occurring molecules derived from natural sources), synthetic biomolecules (i.e. naturally occurring biomolecules that are synthetically prepared and non-naturally occurring molecules or forms of molecules prepared synthetically) or recombinant biomolecules (prepared through the use of recombinant techniques). Examples of biomolecules of interest include, but are not limited to biomolecules disclosed in US 2006/0155384, such as bioadhesives, cell attachment factors, biopolymers, blood proteins, enzymes, extracellular matrix proteins and biomolecules, growth factors and hormones, nucleic acids (DNA and RNA), receptors, synthetic biomolecules, vitamins, drugs, biologically active ions, marker biomolecules etc., including proteins and peptides such as statins and proteins or peptides that stimulate biomineralization and bone formation. Other examples of biomolecules include inorganic, biologically active ions, such as calcium, chromium, fluoride, gold, iodine, iron, potassium, magnesium, manganese, selenium, sulphur, stannous, stannic silver, sodium, zinc, strontium, nitrate, nitrite, phosphate, chloride, sulphate, carbonate, carboxyl or oxide. The biomolecules may e.g. be attached to the surface of the titanium dioxide scaffold via dipping into a solution comprising the biomolecule or via an electrochemical process, such processes being known by the skilled person and e.g. disclosed in WO02/45764 or WO03/086495.

### Method for producing a titanium dioxide scaffold with a nanoporous outer layer

The present document is also directed to a method for producing a titanium dioxide scaffold provided with a nanoporous outer layer comprising titanium dioxide, wherein the pores of said nanoporous outer layer have an average pore diameter of 1 nm-5000 nm, such as 10 nm-1000 nm, 10 nm-500 nm, 50 nm-200 nm or 50 nm-100 nm, said method comprising the steps of:
a) providing a titanium dioxide scaffold,
b) optionally coating at least part of the titanium dioxide scaffold with a titanium dioxide slurry,
c) optionally removing excess slurry from the titanium dioxide scaffold of step b),
d) providing a powder comprising titanium dioxide and at least one polymer onto at least a part of the titanium dioxide scaffold,
e) sintering the titanium dioxide scaffold of step d); and
f) optionally repeating steps b) through e).

In the method for producing a titanium dioxide scaffold with a nanoporous outer layer comprising titanium dioxide, the part of the scaffold which is to be provided with a nanoporous outer layer is provided with a powder comprising titanium dioxide and at least one polymer. Alternatively, at least part of the part of the scaffold to be provided with a nanoporous outer layer is coated with a titanium dioxide slurry (step b)) before being provided with the powder comprising titanium dioxide and at least one polymer in step d). This may e.g. be performed by dipping (immersing) the part(s) of the titanium dioxide scaffold of step a) to be provided with a nanoporous outer layer in the titanium dioxide slurry. Thus, not the whole scaffold has to be coated with a titanium dioxide slurry in step b) when this step is to be performed. Excess titanium dioxide slurry may then be removed from the scaffold such as by carefully centrifuging the scaffold. This centrifugation may e.g. be carried out by a low speed with slow acceleration for 0.5-5 min, 1-5 min, 1-3 min or about 1 min at a speed such as 500-1500 rpm, such as 1300 rpm (based on a rotor size suitable for a Biofuge 22R, Heraeus Sepatec centrifuge).

The titanium dioxide scaffold of step a) is a titanium dioxide scaffold as disclosed elsewhere herein.

The titanium dioxide slurries used in this document both for the preparation of the titnaiumdioxide scaffold and the nanoporous outer layer are typically prepared by dispersing titanium dioxide powder in water. The titanium dioxide powder used may be in the amorphous, anatase, brookit or rutile crystal phase. The titanium dioxide powder may be precleaned with NaOH (e.g. 1 M NaOH) to remove contaminations, such as contaminations of secondary and tertiary phosphates. Alternatively, if titanium dioxide powder free of contaminations of secondary and/or tertiary phosphates is desirable, titanium dioxide powder free of such contaminations is commercially available (e.g. the titanium dioxide from Sachtleben). It may be advantageous to use a titanium dioxide powder having at the most 10 ppm of contaminations of secondary and/or tertiary phosphates. By using titanium dioxide containing less than about 10 ppm of contaminations of secondary and/or tertiary phosphates when preparing the slurry, the titanium dioxide particles are small enough to allow a proper sintering without the addition of organic antiagglomerating compounds and/or surfactants. The titanium dioxide slurries typically have a pH value of about 1.0 to 4.0, preferably about 1.5-2.0, in order to avoid coagulation and to control the viscosity. The pH of the slurry is preferably kept at this pH for the entire duration of dispersion of the titanium dioxide powder in solvent with small additions of HCl (such as 1 M HCl). It is preferable to reduce the size of the titanium dioxide particles as close as possible to the pH value, which gives the theoretical isoelectric point of titanium oxide. For TiO₂ this pH value is 1.7. The mean particle size of the titanium dioxide particles may be 10 µm or less, such as 1.4 µm or less. The titanium oxide particles may be monodispersed. The titanium dioxide powder is typically dispersed in water under stirring and the pH readjusted by the addition of an acid, such as HCl. The stirring may be continued after all titanium dioxide powder is dispersed, such as for about 2-8 hours. The slurry is e.g. dispersed with a rotational dispermat with metal blades, preferably titanium blades. For example the stirring may be performed at a speed of at least 4000 rpm and for at least 2 hours, such as at 5000 rpm for 2 hours or longer. The pH of the slurry is regularly adjusted to the chosen pH value.

The titanium dioxide slurry of step b) typically has a concentration of titanium dioxide of about 2-20 g of TiO₂/ml H₂O.

In step d) of the method, the titanium dioxide scaffold, optionally coated with a titanium dioxide slurry, preferably still wet, is provided with a powder comprising titanium dioxide and at least one polymer onto the surface which is to be provided with the nanoporous outer layer. This may e.g. be performed by dipping the titanium dioxide scaffold in the powder comprising titanium dioxide and at least one polymer. The titanium dioxide scaffold may be wetted at least on the part onto which the nanoporous outer layer is to be provided, e.g. by using an aqueous solution, such as water, e.g. by dipping at least this part of the titanium dioxide scaffold in the aqueous solution. The powder may be spread out in a thin layer before the scaffold is dipped in it. To assure an even coverage of powder on the titanium dioxide scaffold, the part(s) of the scaffold provided with the powder may be rubbed, e.g. by use of a silicone glove. This also removes excess powder and produces an even and thin powder layer on the scaffold surface. The powder comprising titanium dioxide and at least one polymer may be condensed prior to the dipping procedure by mechanical pressing. This may result in a more even thickness and less porous structure of the nanoporous outer layer.

When the titanium dioxide scaffold is coated with a titanium dioxide slurry (step b), it is to be understood that at least part of the surface of the scaffold coated with the titanium dioxide slurry is provided with the powder comprising titanium dioxide and at least one polymer in step d).

The powder comprising titanium dioxide and a polymer of step d) may contain about 2-50 wt%, such as 2-10 wt% or about 10 wt% polymer. A larger amount of polymer relative to titanium dioxide will result in a more porous outer layer.

The polymer may in principle be any polymer, or mixture of two or more polymers, as the polymer will be burnt off during the sintering step e) (see below), thereby forming the pores. However, in order to obtain the desirable ranges of pore diameters, the polymer particle may not have a too large particle diameter as this would result in too large pores, thereby impairing the barrier function of the nanoporous outer layer. The polymer particles therefore typically have a mean particle diameter of 5-250 nm, such as 50-250 nm, e.g. 50-75 nm.

By varying the amount and particle diameter of the polymer, the pore diameter of the nanoporous outer layer may be adjusted to the desired pore diameter.

The polymer typically has a mean polymer molecular weight of 1 000 - 10 000 000 g/mol.

The polymer in the powder comprising titanium dioxide and a polymer of step d) may be selected from the group consisting of acrylonitrile-butadiene-styrene (ABS), allyl resin (allyl), cellulosic, modified natural polymer substance, epoxy, thermoset polyadduct ethylene vinyl alcohol (E/VAL), fluoroplastics (PTFE, FEP, PFA, CTFE, ECTFE, ETFE), ionomer, liquid Crystal Polymer (LCP), melamine formaldehyde (MF), phenol-formaldehyde plastic (PF, phenolic), polyacetal (acetal), polyacrylates (acrylic), polyacrylonitrile (PAN, acrylonitrile), polyamide (PA, nylon), polyamide-imide (PAI), polyaryletherketone (PAEK, Ketone), polybutadiene (PBD), polybutylene (PB), polycarbonate (PC), polydicyclopentadiene (PDCP), polyketone (PK), polyester, polyetheretherketone (PEEK), polyetherimide (PEI), polyethersulfone (PES), polyethylene (PE), polyethylenechlorinates (PEC), polyimide (PI), polymethylpentene (PMP), polyphenylene oxide (PPO), polyphenylene sulfide (PPS), polyphthalamide (PTA), polypropylene (PP), polymer polystyrene (PS), polysulfone (PSU), polyurethane (PU), polyvinylchloride (PVC), polyvinylidene chloride (PVDC), phenol-formaldehyde, polyhexamethylene, poly epoxies, poly phenolics or any co-polymer thereof.

In particular, the polymer may be chosen from the group consisting of polyethylene (PE), polystyrene (PS), polyvinylchloride (PVC), and polypropylene (PP).

The titanium dioxide particles in the powder comprising titanium dioxide and at least one polymer typically has a mean particle diameter of 200 µm or less (but at least 5 nm), e.g. 150 µm or less, 50 µm or less, 1 µm or less, 500 nm or less, 100 nm or less, 50 nm or less, 5 nm-200 µm, 5 nm-150 µm, 5 nm-50 µm, 5 nm-1 µm, 5-500 nm, 5-100 nm, or 5-50 nm.

The sintering step, step e), is typically performed at about 1300 to 1800°C, such as 1500°C, for about 2 hours or more, such as 2-40 hours, such as 30-50 hours, such as 30-40 hours, such as 35-45 hours, or such as about 40 hours. Typically, the sintering is performed at about 1500°C for about 40 hours. During the sintering, the polymer is burnt off, thereby forming the pores. Therefore, the amount and particle diameter of the polymer will affect the pore diameter of the nanoporous outer layer as described elsewhere herein. Also, during sintering the titanium dioxide particles in the nanoporous other layer, which is being formed, fuse and form larger, rounded structures which are believed to be beneficial for osteoblast growth. Also, during the sintering, the titanium dioxide particles of the nanoporous outer layer being formed fuse together with the titanium dioxide of the scaffold, thus attaching the nanoporous outer layer tightly to the titanium dioxide scaffold.

Before providing the titanium dioxide scaffold with the powder comprising titanium dioxide and at least one polymer (steps b)-d) or step d), the titanium dioxide scaffold may be subjected to a procedure of i) providing a titanium dioxide slurry to at least part of the titanium dioxide scaffold, followed by ii) sintering of the titanium dioxide scaffold. This procedure may instead or in addition be performed after performing steps e) or f). It may be preferred to perform this procedure after performing steps e) or f). It is to be understood that at least part of the part of the outer surface of the titanium dioxide scaffold which is to be provided with a nanoporous outer layer is to be provided with the titanium dioxide slurry in this procedure. The titanium dioxide slurry may be provided e.g. by immersion (dipping) in the slurry. The titanium dioxide slurry used in this procedure is typically a highly viscous TiO₂ slurry containing >50wt%, such as 50-80 wt%, TiO₂ dispersed in H₂O. The sintering in this procedure is typically performed at about 1300 to 1800°C, such as 1500°C, for about 2 hours or more, such as 4-50 hours, such as, 10-30 hours, such as 5-20 hours, such as 7-13 hours, such as about 5 hours, 10 hours, 20 hours, 30 hours or 40 hours. Typically, the sintering is performed at about 1500°C for about 10 hours. By performing the procedure of steps i)-ii), the porosity of the nanoporous outer layer will be reduced. Also the surface roughness will change, leading to a surface which is smoother in comparison to the surface of the original titanium dioxide particle.

The titanium oxide scaffold provided in step a) may be prepared by applying a titanium dioxide slurry onto a combustible porous structure, such as a porous polymer structure, burning out the combustible porous structure and sintering the ceramic material obtained after burning out the combustible porous structure. Such a process for producing a titanium dioxide scaffold is disclosed in more detail in WO08078164, which is hereby incorporated by reference. Such a method may include the steps of:
a) preparing a titanium dioxide slurry,
b) providing the titanium dioxide slurry of step a) to a combustible porous structure, such as a polymer sponge structure
c) allowing the slurry to solidify on the combustible porous structure
d) removing the combustible porous structure from the solidified titanium dioxide slurry, wherein step d) may be performed by
   i) slow sintering of the combustible porous structure with the solidified titanium dioxide slurry to about 500°C and holding this temperature for at least 30 minutes,
   ii) fast sintering to about minimum 1500°C or to about 1750°C at ca 3 K/min and holding this temperature for at least 10 hours, and fast cooling to room temperature at at least 3 K/min.

Details regarding the method steps, concentration of titanium dioxide in the slurry etc. for this method is found in WO0807816.

The present document is also directed to a titanium oxide scaffold provided with a nanoporous outer layer comprising titanium dioxide, wherein the pores of said nanoporous outer layer have an average pore diameter of 1 nm-5000 nm, such as 10 nm-1000 nm, 10 nm-500 nm, 50 nm-200 nm or 50 nm-100 nm, obtainable or obtained by the method for producing a nanoporous outer layer on a titanium dioxide scaffold disclosed herein.

### Uses of the titanium dioxide scaffold provided with a nanoporous outer layer comprising titanium dioxide

The titanium dioxide scaffold provided with a nanoporous outer layer comprising titanium dioxide may be implanted into a subject wherein cells will grow into the scaffold structure on the parts of the scaffold not provided with the nanoporous outer layer. It is also possible to seed and grow cells on the titanium dioxide scaffold having a nanoporous outer layer prior to implantation. The interconnected macroporous structure of the titanium dioxide scaffold is especially suitable for tissue engineering, and notably bone tissue engineering, an intriguing alternative to currently available bone repair therapies. In this regard, bone marrow-derived cell seeding of the titanium dioxide scaffold with the nanoporous outer layer is performed using conventional methods, which are well known to those of skill in the art (see e.g. Maniatopoulos *et al.* 1988). Cells are seeded onto the titanium dioxide scaffold with the nanoporous outer layer and cultured under suitable growth conditions. The cultures are fed with media appropriate to establish the growth thereof.

As set out above, cells of various types can be grown throughout the titanium dioxide scaffold. More precisely, cell types include hematopoietic or mesenchymal stem cells, and also include cells yielding cardiovascular, muscular, or any connective tissue. Cells may be of human or other animal origin. However, the titanium dioxide scaffold with the nanoporous outer layer is particularly suited for the growth of osteogenic cells, especially cells that elaborate bone matrix. For tissue engineering, the cells may be of any origin. The cells are advantageously of human origin. A method of growing cells in a titanium dioxide scaffold allows seeded osteogenic cells, for example, to penetrate the titanium dioxide scaffold to elaborate bone matrix, during the *in vitro* stage, with pervasive distribution in the structure of the titanium dioxide scaffold. Osteogenic cell penetration and, as a result, bone matrix elaboration can be enhanced by mechanical, ultrasonic, electric field or electronic means.

The titanium dioxide scaffold provided with a nanoporous outer layer comprising titanium dioxide is useful whenever one is in need of a structure to act as a framework for growth of cells, such as for regeneration of a tissue. The titanium dioxide scaffold with the nanoporous outer layer is particularly useful for the regeneration of bone and cartilage structures. Examples of situations where the regeneration of such structures may be necessary include trauma, surgical removal of bone or teeth or in connection with cancer therapy.

Examples of structures in a subject which wholly or partially may be replaced include, but are not limited to, cranio-facial bones, including arcus zygomaticus, bones of the inner ear (in particular the malleus, stapes and incus), maxillar and mandibular dentoalveolar ridge, walls and floor of eye sockets, walls and floor of sinuses, skull bones and defects in skull bones, socket of hip joint (*Fossa acetabuli*), e.g. in the case of hip joint dysplasias, complicated fractures of long bones including (but not restricted to) humerus, radius, ulna, femur, tibia and fibula, vertebrae, bones of the hands and feet, finger and toe bones, filling of extraction sockets (from tooth extractions), repair of periodontal defects and repair of periimplant defects. In addition the titanium dioxide scaffolds provided with a nanoporous outer layer comprising titanium dioxide are useful for the filling of all types of bone defects resulting from (the removal of) tumors, cancer, infections, trauma, surgery, congenital malformations, hereditary conditions, metabolic diseases (e.g. osteoporosis and diabetes).

The present document is also directed to a titanium dioxide scaffold provided with a nanoporous outer layer comprising titanium dioxide wherein the pores of said nanoporous outer layer have an average pore diameter of 1 nm-5000 nm, such as 10 nm-1000 nm, 10 nm-500 nm, 50 nm-200 nm or 50 nm-100 nm, as defined herein for use as a medical prosthetic device.

This document is therefore also directed to a medical implant, such as an orthopaedic or dental implant or another fixating device, comprising a titanium dioxide scaffold provided with a nanoporous outer layer comprising titanium dioxide wherein the pores of said nanoporous outer layer have an average pore diameter of 1 nm-5000 nm as defined herein. The titanium dioxide scaffold provided with a nanoporous outer layer may be part of a medical implant structure, such as orthopaedic, dental or any other fixating devices or implants. Alternatively, the implant may consist of the titanium dioxide scaffold provided with a nanoporous outer layer comprising or consisting of titanium dioxide.

This document is further directed to the titanium dioxide scaffold comprising a nanoporous outer layer comprising titanium dioxide wherein the pores of said nanoporous outer layer have an average pore diameter of 1 nm-5000 nm or a medical implant comprising such a scaffold for use for the regeneration, repair, substitution and/or restoration of tissue, such as bone.

Also disclosed is a method for the regeneration, repair, substitution and/or restoration of tissue, such as bone, comprising the step of implanting the titanium dioxide scaffold provided with a nanoporous outer layer comprising titanium dioxide wherein the pores of said nanoporous outer layer have an average pore diameter of 1 nm-5000 nm or a medical implant comprising such a scaffold into a subject in need thereof.

Further, this document is directed to the use of the titanium dioxide scaffold comprising a nanoporous outer layer comprising titanium dioxide wherein the pores of said nanoporous outer layer have an average pore diameter of 1 nm-5000 nm, such as 10 nm-1000 nm, 10 nm-500 nm, 50 nm-200 nm or 50 nm-100 nm, or a medical implant comprising such a scaffold for the regeneration, repair, substitution and/or restoration of tissue, such as bone.

The invention will be further described in the following examples, which do not limit the scope of the invention described in the claims.

### EXPERIMENTAL SECTION

### Example 1: Preparation of a cortical wall section on double coated titanium dioxide scaffolds

In order to replicate the dense cortical wall structure of natural bone on the surface of TiO₂ scaffolds, used as artificial bone material, a powder comprising TiO₂ and polyethylen was applied to the same.

A dry mixture of TiO₂ powder (< 100 micron) and polyethylene powder (53-75 micron) in a ratio of 10:1 as by weight was spread out into a thin layer. The titanium dioxide scaffolds, produced by applying a TiO₂-slurry onto a polyurethane foam, burning out the polymer and sintering the ceramic (at 1500 °C for 40 hours), were coated with a new slurry containing 61.5 wt% titanium dioxide. Excess slurry was removed via centrifugation (1300 RPM, slow acceleration, 1 minute). The still wet scaffolds were then dipped in the thin powder layer. To assure an even coverage of powder on the treated surface it was rubbed over with by use of a silicone glove. This also removed excess powder and produced an even and thin layer on the scaffold surface. The scaffolds were then sintered again (40h, 1500°C) in order to consolidate the powder particles to a nanoporous cortical wall and to integrate the cortical wall into the TiO₂ scaffold structure. In this way an even and thin cortical wall like surface with small pores to mimic natural cortical bone was obtained on the scaffold surface. The coating procedure can be repeated if denser/thicker cortical wall is desired. As cross sectional SEM images (Fig. 1) shows, it was possible to fuse a denser barrier, the nanoporous outer layer, on top of the porous scaffold. The TiO₂ particles that were used have adhered and fused together with the porous TiO₂ scaffold. This layer is a few microns thick and can be seen to be much less porous than the titanium dioxide scaffold itself. One can also observe that the PE powder that was blended in the TiO₂ prior to sintering has evaporated and left a nanoporous structure.

### Example 2: Comparison of different ways of producing the nanoporous outer layer

This example shows how it is possible to modulate the pore diameter and porosity of the nanoporous outer layer (cortical wall). Four different procedures where performed: 1) Dipping in dry TiO₂ and polymer powder followed by sintering, 2) Dipping in dry TiO₂ and polymer powder followed by sintering before dipping in highly viscous TiO₂ slurry containing > 50wt% TiO₂ dispersed in HO₂ and sintering, 3) Dipping in pressed dry TiO₂ and polymer powder followed by sintering before dipping highly viscous TiO₂ slurry containing > 50wt% TiO₂ dispersed in H₂O and sintering, 4) dipping in highly viscous TiO₂ slurry containing > 50wt% TiO₂ dispersed in H₂O and sintering followed by dipping in dry TiO₂ and polymer powder. For all experiments, the titanium dioxide scaffold surfaces was wetted by aqueous solution (i.e. only water) and subsequently dipped in a thin layer of TiO₂ powder (particle size < 100 µm) into which small (50-80 µm) PE (polyethylene) particles have been dispersed (ratio of titanium dioxide to polymer is 10:1, based on the weight of the respective substances). All scaffolds were then subjected to sintering (1500 °C for > 2 h) in order to consolidate the prepared cortical wall (nanoporous outer layer) (Fig. 2 (1-4)). The TiO₂ and polymer powder into which the titanium dioxide scaffold was dipped, may be condensed prior to the dipping procedure by mechanical pressing to achieve even thickness and less porous structure for the nanoporous outer layer. The dipping and sintering procedures may be repeated 1-3 times in order to have a cortical wall of desired density and thickness (100-500 µm) and pore diameter of <5 µm.

Some of the cortical walls prepared as described above were then coated with a highly viscous TiO₂ slurry containing > 50wt% TiO₂ dispersed in H₂O. A thin layer of such ceramic slurry was evenly distributed onto the existing denser wall(s) i.e. the cortical walls of the titanium dioxide scaffold(s) so as to reduce large voids in the cortical wall and to provide a smoother surface for osteoblast attachment. Again, the coated scaffolds were then subjected to sintering (1500 °C for > 2 h) in order to consolidate the prepared cortical wall (Fig. 2 (2-3). One can see that both the pore diameter and porosity can be altered by different manufacturing techniques (Fig. 2 (1-4)).

The order of the two procedures described above may also be reversed (Fig. 2(4)).

### Example 3: Growth of osteoblasts on a nanoporous outer layer

Human osteoblast cells were seeded onto the cortical wall (prepared by dipping a titanium dioxide scaffold in pressed dry TiO₂ and polymer powder followed by sintering before dipping in dense TiO₂ slurry and sintering as disclosed in Example 2) at a concentration of 20 000 cells per mL. The cortical wall with the osteoblast cells were kept in DMEM solution for 7 days in an inubactor at 37°C and a 5% CO₂. DMEM solution was exchanged every third day. After cultivation the cortical wall cells were fixed and dried with alcohol. Then the samples were sputter-coated with gold and viewed in SEM as described in Fostad *et al.* 2009. Cells are fairly widespread for a nanoporous outer surface prepared by dipping in pressed dry TiO₂ and polymer powder followed by sintering before dipping in dense TiO₂ slurry and sintering. Holes and edges served as anchor points for the cells, which prevented the osteoblast from entering the underlying porous structure (see Fig. 3).

### Example 4: Effect of polymer particle content on the properties of cortical wall structure

In order to evaluate the effect of polymer particle content of the properties of the cortical wall-like structure, the cortical wall structures presented in Example 1 were produced with varying TiO₂ powder-to-PE particle ratio.

Dry mixtures of TiO₂ powder (< 100 micron) and polyethylene powder (53-75 micron) in a ratio of 10:0, 10:1, and 5:1, 2:1 and 1:1, by weight was spread out into a thin layer. The titanium dioxide scaffolds, produced by applying a TiO₂-slurry onto a polyurethane foam, burning out the polymer and sintering the ceramic (at 1500 °C for 40 hours), were coated with a new slurry containing 61.5 wt% titanium dioxide. Excess slurry was removed via centrifugation (1300 RPM, slow acceleration, 1 minute). The still wet scaffolds were then dipped in the thin powder layer. To assure an even coverage of powder on the treated surface it was rubbed over with by use of a silicone glove. This also removed excess powder and produced an even and thin layer on the scaffold surface. The scaffolds were then sintered again (40h, 1500°C) in order to consolidate the powder particles to a nanoporous cortical wall and to integrate the cortical wall into the TiO₂ scaffold structure. As shown in Fig. 4, the polymer particle content influenced the morphology of the cortical wall structure. As the ratio of the PE particles increased in the powder mixture, the homogeneity of the pore network formed by the fused TiO₂ particles after the PE particles had evaporated reduced markedly, while porosity of the cortical wall structure increased. This less inhomogenous pore distribution is considered to reduce the capacity of the cortical wall structure to inhibit soft tissue ingrowth into the scaffold structure. The use of TiO₂-to-polymer ratio 1:1 led to no formation of a cortical wall due to the large polymer content in the unsintered cortical wall. Following the evaporation of the polymer particles, the loosely packed TiO₂ particles remained too far apart from each other to fused together to form the wall structure. Furthermore, the absence of the polymer particles (10:0 ratio) led to less homogenous distribution of the nano- and micropores in the cortical wall structure in comparison to the 10:1 TiO₂-to-polymer ratio, and the pore network was less connected when no PE particles were added into the TiO₂ powder. The three-dimensional structure of cortical wall structure prepared using a TiO₂ to polymer ratio of 10:1 is shown in Fig. 5.

### Example 5:

Scaffolds as described in example 1 were placed in lateral augmentation in mini pig jaws. The premolar , P1-4 was removed 14 weeks prior to surgery. The cortical bone was trimmed with a trephan burr, and fixed with two titanium screws. Negative control was empty site. After six months of healing there was substantially more bone on the cortical wall (Fig. 6) in comparison to sham. The evaluation was performed with microCT (Skycan 1172, Bruker, Kontich, Belgium) and histology.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Unless expressly described to the contrary, each of the preferred features described herein can be used in combination with any and all of the other herein described preferred features.

### REFERENCES

Brezny R, Green DJ, Dam CQ. Evaluation of strut strength in open-cell ceramics. J Am Ceram Soc 1989;72:885-889.
G. Fostad, B. Hafell, A. Førde, R. Dittmann, R. Sabetrasekh, J. Will, J.E. Ellingsen, S.P. Lyngstadaas, H.J. Haugen, Loadable TiO2 scaffolds. A correlation study between processing parameters, micro CT analysis and mechanical strength, Journal of the European Ceramic Society, Volume 29, Issue 13, October 2009, Pages 2773-2781, ISSN 0955-2219, 10.1016/j.jeurceramsoc.2009.03.017.)
Larry S., Liebovitch, Tibor Toth, A fast algorithm to determine fractal dimensions by box counting, Physics Letters A, Volume 141, Issues 8-9, 20 November 1989, Pages 386-390, ISSN 0375-9601, http://dx.doi.org/10.1016/0375-9601(89)90854-2. (http://www.sciencedirect.com/science/article/pii/0375960189908542)

## Claims

1. A titanium dioxide scaffold, wherein at least part of the outer surface of said titanium dioxide scaffold is provided with a nanoporous outer layer comprising titanium dioxide, wherein the pores of said nanoporous outer layer have an average pore diameter of 1 nm-5000 nm, such as 10 nm-1000 nm.

2. The titanium dioxide scaffold according to claim 1, wherein said nanoporous outer layer has a thickness of 10-1000 µm, such as 50-500 µm.

3. The titanium dioxide scaffold of claim 1 or 2, wherein said nanoporous outer layer has a porosity of 1-50%, such as 3 -25%.

4. A method for producing a titanium dioxide scaffold as defined in any one of the preceding claims, said method comprising the steps of:
a) providing a titanium dioxide scaffold,
b) optionally coating at least part of the titanium dioxide scaffold with a titanium dioxide slurry,
c) optionally removing excess slurry from the titanium dioxide scaffold of step b), such as by centrifugation,
d) providing a powder comprising titanium dioxide and at least one polymer onto at least a part of the outer surface of the titanium dioxide scaffold,
e) sintering the titanium dioxide scaffold of step d); and
f) optionally repeating steps b) through e).

5. The method according to claim 4, wherein step b) is preceded by providing a titanium dioxide slurry to at least part of the titanium dioxide scaffold, followed by sintering the titanium dioxide scaffold.

6. The method according to claim 4 or 5, wherein step e) or f) is followed by providing a titanium dioxide slurry to at least part of the titanium dioxide scaffold, followed by sintering the titanium dioxide scaffold.

7. The method according to any one of claims 4-6, wherein step e) is performed at about 1300 to 1800°C for about 2 hours or more, such as 30-50 hours, such as about 1500°C for 40 hours.

8. The method according to any of claims 4-7, wherein said powder comprising titanium dioxide and at least one polymer of step d) contains 2-50 wt% polymer, such as 2-10 wt% polymer, such as 10 wt% polymer,

9. The method according to any of claims 4-8, wherein said polymer of said powder comprising titanium dioxide and at least one polymer of step d) has a mean particle diameter of 5-250 nm such as 50-250 nm.

10. The method according to any one of claims 4-9, wherein the titanium oxide scaffold provided in step a) is prepared by applying a titanium dioxide slurry onto a porous polymer structure, burning out the porous polymer structure and sintering the ceramic material obtained after burning out the porous polymer structure.

11. A titanium oxide scaffold provided with a nanoporous outer layer comprising titanium dioxide obtainable or obtained by the method of any one of claims 4-10.

12. A medical implant comprising a titanium dioxide scaffold as defined in any one of claims 1-3 or 11.

13. A titanium dioxide scaffold as defined in any one of claims 1-3 or 11 or a medical implant as defined in claim 12 for use for the regeneration, repair, substitution and/or restoration of tissue, such as bone.

## Patentansprüche

1. Titandioxidgerüst, wobei wenigstens ein Teil der äußeren Oberfläche des Titandioxidgerüsts mit einer nanoporösen äußeren Schicht, die Titandioxid umfasst, versehen ist, wobei die Poren der nanoporösen äußeren Schicht einen durchschnittlichen Porendurchmesser von 1 nm - 5.000 nm, zum Beispiel 10 nm - 1.000 nm, haben.

2. Titandioxidgerüst gemäß Anspruch 1, wobei die nanoporöse äußere Schicht eine Dicke von 10-1.000 µm, zum Beispiel 50-500 µm, hat.

3. Titandioxidgerüst gemäß Anspruch 1 oder 2, wobei die nanoporöse äußere Schicht eine Porosität von 1-50%, zum Beispiel 3-25%, hat.

4. Verfahren zur Herstellung eines Titandioxidgerüsts, wie es in einem der vorangehenden Ansprüche definiert ist, wobei das Verfahren die folgenden Schritte umfasst:
a) Bereitstellen eines Titandioxidgerüsts,
b) gegebenenfalls wenigstens teilweise Beschichten des Titandioxidgerüsts mit einer Titandioxidaufschlämmung,
c) gegebenenfalls Entfernen einer überschüssigen Aufschlämmung von dem Titandioxidgerüst von Schritt b), zum Beispiel durch Zentrifugation,
d) Bereitstellen eines Pulvers, das Titandioxid und wenigstens ein Polymer umfasst, auf wenigstens einem Teil der äußeren Oberfläche des Titandioxidgerüsts,
e) Sintern des Titandioxidgerüsts von Schritt d) und
f) gegebenenfalls Wiederholen der Schritte b) bis e).

5. Verfahren gemäß Anspruch 4, wobei Schritt b) ein Bereitstellen einer Titandioxidaufschlämmung auf wenigstens einem Teil des Titandioxidgerüsts vorausgeht, gefolgt von einem Sintern des Titandioxidgerüsts.

6. Verfahren gemäß Anspruch 4 oder 5, wobei auf Schritt e) oder f) Bereitstellen einer Titandioxidaufschlämmung auf wenigstens einem Teil des Titandioxidgerüsts folgt, gefolgt von einem Sintern des Titandioxidgerüsts.

7. Verfahren gemäß einem der Ansprüche 4-6, wobei Schritt e) bei etwa 1.300 bis 1.800°C für etwa 2 Stunden oder mehr, zum Beispiel 30-50 Stunden, zum Beispiel etwa 1.500°C für 40 Stunden, durchgeführt wird.

8. Verfahren gemäß einem der Ansprüche 4-7, wobei das Pulver, das Titandioxid und wenigstens ein Polymer umfasst, von Schritt d) 2-50 Gew.-% Polymer, zum Beispiel 2-10 Gew.-% Polymer, zum Beispiel 10 Gew.-% Polymer, enthält.

9. Verfahren gemäß einem der Ansprüche 4-8, wobei das Polymer des Pulvers, das Titandioxid und wenigstens ein Polymer umfasst, von Schritt d) einen mittleren Partikeldurchmesser von 5-250 nm, zum Beispiel 50-250 nm, hat.

10. Verfahren gemäß einem der Ansprüche 4-9, wobei das Titanoxidgerüst, das in Schritt a) bereitgestellt wird, hergestellt wird, indem eine Titandioxidaufschlämmung auf eine poröse Polymerstruktur aufgetragen wird, die poröse Polymerstruktur ausgebrannt wird und das nach Ausbrennen der porösen Polymerstruktur erhaltene keramische Material gesintert wird.

11. Titanoxidgerüst, das mit einer nanoporösen äußeren Schicht, die Titandioxid umfasst, versehen ist, erhältlich oder erhalten durch das Verfahren gemäß einem der Ansprüche 4-10.

12. Medizinisches Implantat, das ein Titandioxidgerüst, wie es in einem der Ansprüche 1-3 oder 11 definiert ist, umfasst.

13. Titandioxidgerüst, wie es in einem der Ansprüche 1-3 oder 11 definiert ist, oder medizinisches Implantat, wie es in Anspruch 12 definiert ist, zur Verwendung für die Regeneration, Reparatur, Substitution und/oder Wiederherstellung von Gewebe, zum Beispiel Knochen.

## Revendications

1. Échafaudage de dioxyde de titane, dans lequel au moins une partie de la surface externe dudit échafaudage de dioxyde de titane est pourvue d'une couche externe nanoporeuse comprenant du dioxyde de titane, les pores de ladite couche externe nanoporeuse ayant un diamètre de pores moyen de 1 nm à 5000 nm, tel que de 10 nm à 1000 nm.

2. Échafaudage de dioxyde de titane selon la revendication 1, dans lequel ladite couche externe nanoporeuse a une épaisseur de 10 à 1000 µm, telle que de 50 à 500 µm.

3. Échafaudage de dioxyde de titane selon la revendication 1 ou 2, dans lequel ladite couche externe nanoporeuse a une porosité de 1 à 50 %, telle que de 3 à 25 %.

4. Procédé pour produire un échafaudage de dioxyde de titane tel que défini selon l'une quelconque des revendications précédentes, ledit procédé comprenant les étapes consistant à :
a) fournir un échafaudage de dioxyde de titane,
b) revêtir facultativement au moins une partie de l'échafaudage de dioxyde de titane d'une suspension de dioxyde de titane,
c) ôter facultativement l'excès de suspension de l'échafaudage de dioxyde de titane de l'étape b), par exemple par centrifugation,
d) prévoir une poudre comprenant du dioxyde de titane et au moins un polymère sur au moins une partie de la surface externe de l'échafaudage de dioxyde de titane,
e) fritter l'échafaudage de dioxyde de titane de l'étape d) ; et
f) répéter facultativement les étapes b) à e).

5. Procédé selon la revendication 4, dans lequel l'étape b) est précédée par la fourniture d'une suspension de dioxyde de titane sur au moins une partie de l'échafaudage de dioxyde de titane, suivie par un frittage de l'échafaudage de dioxyde de titane.

6. Procédé selon la revendication 4 ou 5, dans lequel l'étape e) ou f) est suivie de la fourniture d'une suspension de dioxyde de titane sur au moins une partie de l'échafaudage de dioxyde de titane, suivie par un frittage de l'échafaudage de dioxyde de titane.

7. Procédé selon l'une quelconque des revendications 4 à 6, dans lequel l'étape e) est réalisée à une température d'environ 1300 C à 1800°C pendant environ 2 heures ou plus, par exemple 30 à 50 heures, telle qu'une température d'environ 1500 C pendant 40 heures.

8. Procédé selon l'une quelconque des revendications 4 à 7, dans lequel ladite poudre comprenant du dioxyde de titane et au moins un polymère de l'étape d) renferme de 2 à 50 % en poids de polymère, par exemple de 2 à 10 % en poids de polymère, tel que 10 % en poids de polymère.

9. Procédé selon l'une quelconque des revendications 4 à 8, dans lequel ledit polymère de ladite poudre comprenant du dioxyde de titane et au moins un polymère de l'étape d) a un diamètre de particules moyen de 5 nm à 250 nm, tel que de 50 nm à 250 nm.

10. Procédé selon l'une quelconque des revendications 4 à 9, dans lequel l'échafaudage de dioxyde de titane fourni à l'étape a) est préparé en appliquant une suspension de dioxyde de titane sur une structure polymère poreuse, en calcinant la structure polymère poreuse et en frittant le matériau céramique obtenu après calcination de la structure polymère poreuse.

11. Échafaudage de dioxyde de titane pourvu d'une couche externe nanoporeuse comprenant du dioxyde de titane pouvant être obtenu ou étant obtenu par le procédé selon l'une quelconque des revendications 4 à 10.

12. Implant médical comprenant un échafaudage de dioxyde de titane tel que défini selon l'une quelconque des revendications 1 à 3 ou 11.

13. Échafaudage de dioxyde de titane tel que défini selon l'une quelconque des revendications 1 à 3 ou 11 ou implant médical selon la revendication 12 destiné à être utilisé pour la régénération, la réparation, la substitution et/ou la restauration de tissu, par exemple de tissu osseux.
